# EUROPEAN PATENT APPLICATION

(11) **EP 2 204 117 A1**
(43) Date of publication of application: **07.07.2010**
(21) Application number: 08837408.7
(22) Date of filing: 19.08.2008
(51) Int. Cl.: A61B 1/00, A61B 5/07

(54) **LIVING BODY INFORMATION ACQUIRING DEVICE, LIVING BODY OBSERVING SYSTEM, AND METHOD OF DRIVING LIVING BODY OBSERVING SYSTEM**

(30) Priority: 09.10.2007 JP 2007263700
(71) Applicant: Olympus Corporation, Tokyo 151-0072 (JP)
(72) Inventor: YOSHIZAWA, Fukashi, Tokyo 151-0072 (JP)
(74) Representative: Röss, Walter Josef Alfred
(86) International application number: PCT/JP2008/064735
(87) International publication number: WO 2009/047946

(57) **Abstract**

A living body information acquiring apparatus according to the present invention includes: a living body information acquiring section for acquiring living body information in a living body; a wireless transmission section for wirelessly transmitting the living body information to outside of the living body; a power section for supplying driving power of the living body information acquiring section and the wireless transmission section; a magnetic field detecting section for detecting a magnetic field from outside and outputting a detection result as an electric signal; and a power supply control section for controlling a supply state of the driving power supplied from the power section to the living body information acquiring section and the wireless transmission section based on the electric signal.

## Description

### Technical Field

The present invention relates to a living body information acquiring apparatus, a living body observation system and a method of driving a living body observation system. More particularly, the present invention relates to a living body information acquiring apparatus, a living body observation system and a method of driving a living body observation system including a power section having a battery or the like.

### Background Art

Conventionally, endoscopes have been widely used in a medical field or the like. In particular, endoscopes in a medical field are mainly used for the purpose of observing inside of a living body. One type of the endoscopes described above that has been proposed in recent years is a capsule endoscope that is swallowed by a test subject so that the capsule endoscope is disposed in a body cavity, the capsule endoscope capable of picking up images of subjects while moving along the body cavity through peristaltic movement, and wirelessly transmitting the picked-up images of the subjects to outside as an image pickup signal.

Japanese Patent Application Laid-Open Publication No. 2001-224553 proposes an apparatus having substantially the same functions as those of the capsule endoscope described above, for example.

Japanese Patent Application Laid-Open Publication No. 2001-224553 describes a capsule endoscope having a configuration in which a reed switch whose contacts open when placed in a magnetic field is used as a non-contact power switch. The capsule endoscope described in Japanese Patent Application Laid-Open Publication No. 2001-224553 includes the reed switch described above and is thereby configured such that the contacts of the reed switch open to power OFF the capsule endoscope when the capsule endoscope is stored in a container or a storage case having a magnet, and the contacts of the reed switch close to power ON the capsule endoscope when the capsule endoscope is removed from the container or the storage case, for example.

However, the capsule endoscope disclosed in Japanese Patent Application Laid-Open Publication No. 2001-224553 is configured such that the power is turned on when the capsule endoscope is removed from the container or storage case having a magnet. Therefore, an internal battery starts to be consumed at a stage before the capsule endoscope is arranged in a living body. As a result, the capsule endoscope disclosed in the Japanese Patent Application Laid-Open Publication No. 2001-224553 has a problem in that, before the capsule endoscope reaches a desired region, the remaining battery level decreases to a level at which image pickup of the desired region is impossible and observation of the desired region cannot be performed.

Furthermore, in order to turn off the power of the capsule endoscope disclosed in the Japanese Patent Application Laid-Open publication No. 2001-224553 again after the power was turned on once, it is necessary to apply a magnetic field, the direction of which is set in accordance with the direction of the reed switch and a strength of which is stronger than a predetermined strength, to the capsule endoscope while using a permanent magnet and the like. That is, the capsule endoscope disclosed in Japanese Patent Application Laid-Open Publication No. 2001-224553 has a problem in that troublesome operation is required for turning off the power again after the power was turned on once.

The present invention has been achieved in view of the above-described circumstances, and an object of the present invention is to provide a living body information acquiring apparatus, a living body observation system, and a method of driving a living body observation system which are capable of easily switching between ON and OFF of a power and suppressing consumption of an internal battery, compared with a conventional apparatus and the like.

### Disclosure of Invention

### Means for Solving the Problem

A living body information acquiring apparatus according to the present invention includes: a living body information acquiring section for acquiring living body information in a living body; a wireless transmission section for wirelessly transmitting the living body information to outside of the living body; a power section for supplying driving power of the living body information acquiring section and the wireless transmission section; a magnetic field detecting section for detecting a magnetic field from outside and outputting a detection result as an electric signal; and a power supply control section for controlling a supply state of the driving power supplied from the power section to the living body information acquiring section and the wireless transmission section based on the electric signal.

A living body observation system according to the present invention includes: a living body information acquiring apparatus including a living body information acquiring section for acquiring living body information in a living body, a wireless transmission section for wirelessly transmitting the living body information to outside of the living body, a power section for supplying driving power of the living body information acquiring section and the wireless transmission section, a magnetic field detecting section for detecting a magnetic field from outside and outputting a detection result as an electric signal, and a power supply control section for controlling a supply state of the driving power supplied from the power section to the living body information acquiring section and the wireless transmission section based on the electric signal; and a magnetic field generating section for generating an alternating-current magnetic field outside the living body information acquiring apparatus.

A method of driving a living body observation system according to the present invention is a method of driving a living body observation system including at least: a living body information acquiring apparatus including a living body information acquiring section for acquiring living body information in a living body, a wireless transmission section for wirelessly transmitting the living body information to outside of the living body, a power section for supplying driving power of the living body information acquiring section and the wireless transmission section, a magnetic field detecting section for detecting a magnetic field from outside and outputting a detection result as an electric signal, and a power supply control section for controlling a supply state of the driving power supplied from the power section to the living body information acquiring section and the wireless transmission section based on the electric signal; and a magnetic field generating section for generating an alternating-current magnetic field outside the living body information acquiring apparatus, wherein the living body information acquiring apparatus is switched to a power ON state or a power OFF state in response to an alternating-current magnetic field intermittently generated from the magnetic field generating section.

A method of driving a living body observation system according to the present invention is a method of driving a living body observation system including at least: a living body information acquiring apparatus including a living body information acquiring section for acquiring living body information in a living body, a wireless transmission section for wirelessly transmitting the living body information to outside of the living body, a power section for supplying driving power of the living body information acquiring section and the wireless transmission section, a magnetic field detecting section for detecting a magnetic field from outside and outputting a detection result as an electric signal, and a power supply control section for controlling a supply state of the driving power supplied from the power section to the living body information acquiring section and the wireless transmission section based on the electric signal; and a magnetic field generating section for generating an alternating-current magnetic field outside the living body information acquiring apparatus, wherein the living body information acquiring apparatus is powered ON only during a period when the magnetic field generating section generates an alternating-current magnetic field.

A method of driving a living body observation system according to the present invention is a method of driving a living body observation system including at least: a living body information acquiring apparatus including a living body information acquiring section for acquiring living body information in a living body, a wireless transmission section for wirelessly transmitting the living body information to outside of the living body, a power section for supplying driving power of the living body information acquiring section and the wireless transmission section, a magnetic field detecting section for detecting a magnetic field from outside and outputting a detection result as an electric signal, and a power supply control section for controlling a supply state of the driving power supplied from the power section to the living body information acquiring section and the wireless transmission section based on the electric signal; and a magnetic field generating section for generating an alternating-current magnetic field outside the living body information acquiring apparatus, wherein the living body information acquiring apparatus is switched to a power ON state or a power OFF state every time the magnetic field generating section generates an alternating-current magnetic field.

A method of driving a living body observation system according to the present invention is a method of driving a living body observation system including at least: a living body information acquiring apparatus including a living body information acquiring section for acquiring living body information in a living body, a wireless transmission section for wirelessly transmitting the living body information to outside of the living body, a power section for supplying driving power of the living body information acquiring section and the wireless transmission section, a magnetic field detecting section for detecting a magnetic field from outside and outputting a detection result as an electric signal, and a power supply control section for controlling a supply state of the driving power supplied from the power section to the living body information acquiring section and the wireless transmission section based on the electric signal; and a magnetic field generating section for generating an alternating-current magnetic field outside the living body information acquiring apparatus, wherein the living body information acquiring apparatus is switched to a power ON state or a power OFF state in response to an alternating-current magnetic field whose frequency gradually changes, the alternating-current magnetic field being intermittently generated from the magnetic field generating section.

### Brief Description of the Drawings

Fig. 1 illustrates a configuration of a main portion of a living body observation system according to a first embodiment of the present invention;
Fig. 2 illustrates an example of a specific configuration of a power supply section and a magnetic field detecting section according to the first embodiment of the present invention;
Fig. 3 illustrates a correlation between operations of a power supply section and a magnetic field detecting section, and a power state of a capsule endoscope according to the first embodiment of the present invention;
Fig. 4 illustrates an example of a specific configuration of a power supply section and a magnetic field detecting section according to a second embodiment of the present invention;
Fig. 5 illustrates a correlation between operations of a power supply section and a magnetic field detecting section, and a power state of a capsule endoscope according to the second embodiment of the present invention; and
Fig. 6 illustrates a correlation between operations of a power supply section and a magnetic field detecting section, and a power state of a capsule endoscope according to a third embodiment of the present invention.

### Best Mode for Carrying Out the Invention

In the following, embodiments of the present invention will be described with reference to the drawings.

### (First Embodiment)

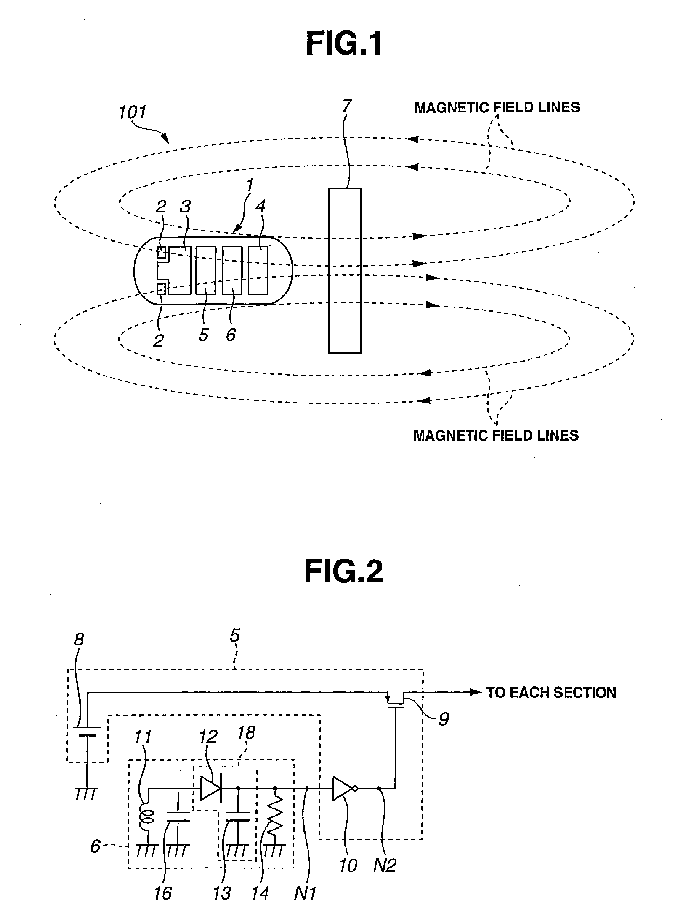

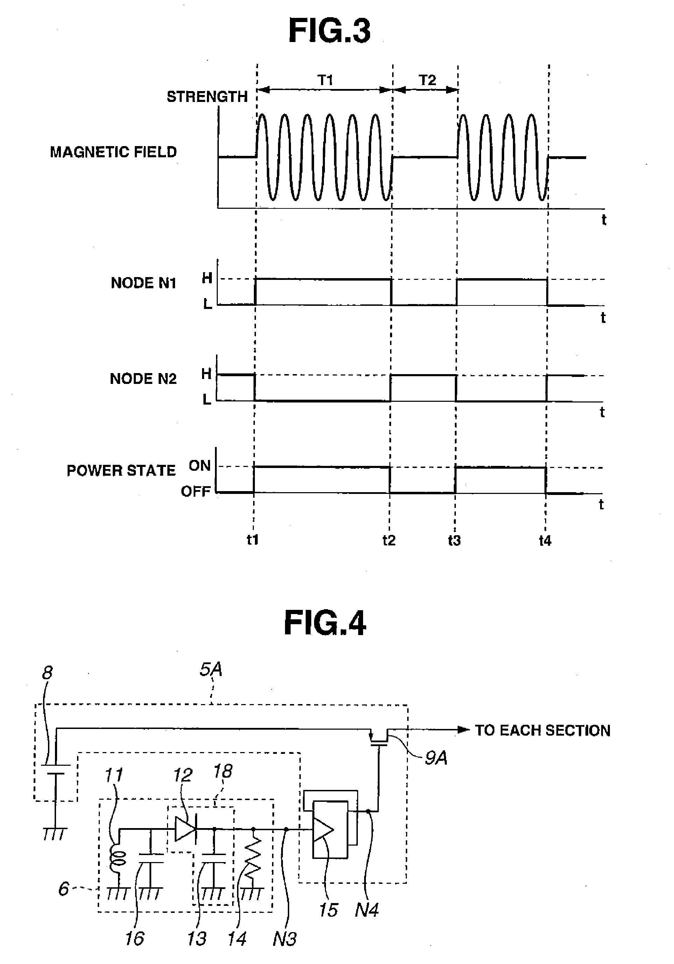

Figs. 1 to 3 relate to a first embodiment of the present invention. Fig. 1 illustrates a configuration of a main portion of a living body observation system according to the first embodiment. Fig. 2 illustrates an example of a specific configuration of a power supply section and a magnetic field detecting section according to the first embodiment. Fig. 3 illustrates a correlation between operations of the power supply section and the magnetic field detecting section, and a power state of a capsule endoscope according to the first embodiment.

As shown in Fig. 1, a living body observation system 101 includes a capsule endoscope 1 having dimensions and a shape to be disposed inside a living body, and a magnetic field generating section 7 for generating an alternating-current magnetic field outside the capsule endoscope 1.

The magnetic field generating section 7 has a configuration in which a magnetic-field generating state can be switched to ON or OFF according to an operation of an unillustrated switch or the like by a user, for example.

The capsule endoscope 1 includes therewithin an illuminating section 2 for emitting light for illuminating a subject inside a living body, an image pickup section 3 for picking up an image of the subject illuminated by the illuminating section 2 and outputting the image as an image pickup signal, a wireless transmission section 4 for wirelessly transmitting the image pickup signal outputted from the image pickup section 3 to outside of the living body, a power supply section 5 for supplying driving power required for driving each of the illuminating section 2, the image pickup section 3 and the wireless transmission section 4, and a magnetic field detecting section 6 capable of detecting the alternating-current magnetic filed generated in the magnetic field generating section 7 as shown in Fig. 1.

That is, the illuminating section 2 and the image pickup section 3 constitute a living body information acquiring section in the present embodiment.

The power supply section 5 includes a power section 8 having a battery or the like, a P-channel FET 9, and an inverter 10 for inverting an outputted signal from the magnetic field detecting section 6 as shown in Fig. 2.

The P-channel FET 9 having functions as a power supply control section and a switch section has its source connected to the power section 8, its gate connected to an output end of the inverter 10 and its drains respectively connected to the illuminating section 2, the image pickup section 3, and the wireless transmission section 4.

The power supply section 5 is not limited to the configuration using the P-channel FET 9. The power supply section 5 may also be constituted by an electronic switch or the like having a similar switching function.

The magnetic field detecting section 6 includes a magnetic field detecting coil 11 for outputting an electric signal corresponding to the alternating-current magnetic field generated in the magnetic field generating section 7, a rectifying section 18 for rectifying and outputting the electric signal outputted from the magnetic field detecting coil 11, a resistor 14, and a resonant capacitor 16.

The magnetic field detecting coil 11 may be formed of a solenoid coil or a planar coil, for example. So long as the magnetic field detecting coil 11 can be disposed in the capsule endoscope 1, the magnetic field detecting coil 11 may have any shape.

The rectifying section 18 has a diode 12 whose input end is connected to an output end of the magnetic field detecting coil 11, and a smoothing capacitor 13 for smoothing an electric signal outputted from the diode 12. The rectifying section 18 in the present embodiment may not be limited to one for performing half-wave rectification but may be one for performing full-wave rectification.

The resistor 14 is connected to an output end of the diode 12 in parallel with the smoothing capacitor 13.

The resonant capacitor 16 is connected to the input end of the diode 12 in parallel with the magnetic field detecting coil 11.

Here, the operations of the power supply section 5 and the magnetic field detecting section 6 in the present embodiment will be described.

First, when the magnetic field generating section 7 generates an alternating-current magnetic field at timing of time t1, a potential difference is generated between both ends of the magnetic field detecting coil 11 by electromagnetic induction. Also, an alternating-current electric signal corresponding to the potential difference is outputted to the rectifying section 18.

The alternating-current electric signal outputted from the magnetic field detecting coil 11 is rectified in the rectifying section 18. The alternating-current electric signal is thereby converted into a direct-current electric signal and is outputted to an input end of the inverter 10. At this time, a signal level at a node N1 on the input end side of the inverter 10 becomes a high (referred to as H below) level to cause a signal level at a node N2 on the output end side of the inverter 10 to become a low (referred to as L below) level as shown in Fig. 3.

When the signal level at the node N2 becomes the L level, the P-channel FET 9 becomes an ON state. The power section 8 thus starts to supply driving power to each of the illuminating section 2, the image pickup section 3, and the wireless transmission section 4 to power ON the capsule endoscope 1.

After that, when the magnetic field generating section 7 stops generating the alternating-current magnetic field at timing of time t2, an electric charge accumulated in the smoothing capacitor 13 is discharged via the resistor 14. Accordingly, the signal level at the node N1 becomes the L level and the signal level at the node N2 becomes the H level as shown in Fig. 3.

When the signal level at the node N2 becomes the H level, the P-channel FET 9 becomes an OFF state. The power section 8 thus stops supplying the driving power to each of the illuminating section 2, the image pickup section 3, and the wireless transmission section 4 to power OFF the capsule endoscope 1.

Thereafter, when the magnetic field generating section 7 generates an alternating-current magnetic field again at timing of time t3, the capsule endoscope 1 is powered ON by the above operations, and the same operations will be repeated.

That is, the capsule endoscope 1 of the present embodiment has such a configuration that the capsule endoscope 1 is powered ON during a period T1 when the magnetic field generating section 7 generates an alternating-current magnetic field, and the capsule endoscope 1 is powered OFF during a period T2 when the magnetic field generating section 7 does not generate an alternating-current magnetic field.

The magnetic field detecting coil 11 and the resonant capacitor 16 constitute a resonant circuit. Therefore, in the present embodiment, it is possible to improve detection sensitivity to the alternating-cunent magnetic field generated from the magnetic field generating section 7, and also, to lower detection sensitivity to an unintended disturbance magnetic field by adjusting a resonant frequency of the resonant circuit to a frequency of the alternating-current magnetic field generated from the magnetic field generating section 7. As a result, stable control is achieved such that the capsule endoscope 1 can be reliably switched between ON and OFF.

The capsule endoscope 1 of the present embodiment may further include a limiter circuit, for example, as a configuration for suppressing an excessive rise in potential of the node N1.

Next, observation or the like using the capsule endoscope 1 which performs the above operations will be described.

First, a user removes the capsule endoscope 1 stored in a container or a storage case having no magnet.

The user powers ON the capsule endoscope 1 by the alternating-current magnetic field generated from the magnetic field generating section 7, and checks the operation of the capsule endoscope 1. Then, the capsule endoscope 1 is taken orally to be disposed inside a body of a test subject.

The present embodiment is not limited to the configuration in which the capsule endoscope 1 is powered ON after being removed from the container or the storage case. The capsule endoscope 1 may be also powered ON by applying the alternating-current magnetic field to the capsule endoscope 1 which is being stored in the container or the storage case, for example.

Also, in the present embodiment, it is possible to keep the capsule endoscope 1 powered ON and appropriately switch the capsule endoscope 1 between ON and OFF by the alternating-current magnetic field generated from the magnetic field generating section 7 after disposing the capsule endoscope 1 in the body of the test subject. To be more specific, it is possible to perform control (or operation) such that the capsule endoscope 1 is powered OFF by stopping the generation of the alternating-current magnetic field in the magnetic field generating section 7 when the capsule endoscope 1 is passing through a region which is not required to be observed or the like, and the capsule endoscope 1 is powered ON by generating the alternating-current magnetic field from the magnetic field generating section 7 when the capsule endoscope 1 reaches a desired observation region, for example.

As described above, the living body observation system 101 of the present embodiment has a configuration in which the capsule endoscope 1 can be easily switched between ON and OFF at a user-desired timing. Accordingly, the living body observation system 101 of the present embodiment can reduce draining of the internal battery in comparison with conventional systems, and also, can more reliably observe a desired region.

### (Second Embodiment)

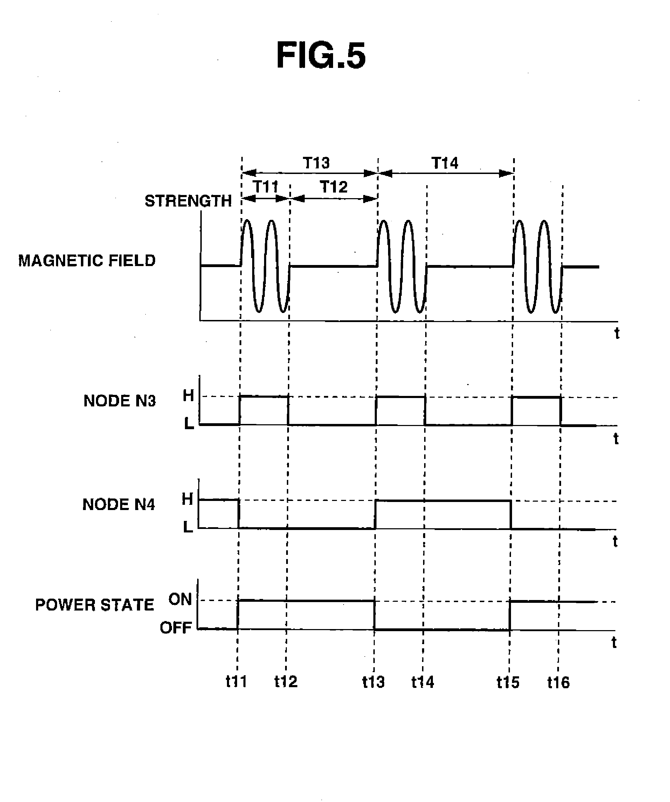

Figs. 4 and 5 relate to a second embodiment of the present invention. Fig. 4 illustrates an example of a specific configuration of a power supply section and a magnetic field detecting section according to the second embodiment. Fig. 5 illustrates a correlation between operations of the power supply section and the magnetic field detecting section, and a power state of a capsule endoscope according to the second embodiment.

In the following description, the detailed description of portions having the same configurations as those of the first embodiment will be omitted. In the present embodiment, portions different from those of the first embodiment will be mainly described.

A power supply section 5A in the present embodiment includes the power section 8, a frequency divider circuit 15 for dividing an outputted signal from the magnetic field detecting section 6 by two, and a P-channel FET 9A as shown in Fig. 4.

The P-channel FET 9A having functions as a power supply control section and a switch section has its source connected to the power section 8, its gate connected to an output end of the frequency divider circuit 15, and its drains respectively connected to the illuminating section 2, the image pickup section 3, and the wireless transmission section 4.

Here, the operations of the power supply section 5A and the magnetic field detecting section 6 in the present embodiment will be described.

First, when the magnetic field generating section 7 generates an alternating-current magnetic field at timing of time t11, a potential difference is generated between both ends of the magnetic field detecting coil 11 by electromagnetic induction. Also, an alternating-current electric signal corresponding to the potential difference is outputted to the rectifying section 18.

The alternating-current electric signal outputted from the magnetic field detecting coil 11 is rectified in the rectifying section 18. The alternating-current electric signal is thereby converted into a direct-current electric signal and is outputted to an input end of the frequency divider circuit 15. At this time, a signal level at a node N3 on the input end side of the frequency divider circuit 15 becomes an H level as shown in Fig. 5. After that, when the magnetic field generating section 7 stops generating the alternating-current magnetic field at timing of time t12, an electric charge accumulated in the smoothing capacitor 13 is discharged via the resistor 14. The signal level at the node N3 thereby becomes an L level as shown in Fig. 5.

That is, the signal level at the node N3 becomes the H level during a period T11 when the magnetic field generating section 7 generates an alternating-current magnetic field, and becomes the L level during a period T12 when the magnetic field generating section 7 does not generate an alternating-current magnetic field.

On the other hand, a signal level at a node N4 on the output end side of the frequency divider circuit 15 becomes an L level during a period from time t11 to time t13 (period T13) shown in Fig. 5, and becomes an H level during a period from time t13 to time t15 (after passing through time t14) (period T14) shown in Fig. 5 corresponding to the outputted signal from the magnetic field detecting section 6.

When the signal level at the node N4 becomes the L level, the P-channel FET 9A becomes an ON state. The power section 8 thus starts to supply driving power to each of the illuminating section 2, the image pickup section 3, and the wireless transmission section 4 to power ON the capsule endoscope 1 during the period from time t11 to time t13 (that is, the period T13). Also, when the signal level at the node N4 becomes the H level, the P-channel FET 9A becomes an OFF state. The power section 8 thus stops supplying the driving power to each of the illuminating section 2, the image pickup section 3, and the wireless transmission section 4 to power OFF the capsule endoscope 1 during the period from time t13 to time t15 (that is, the period T14).

The description of observation or the like using the capsule endoscope 1 of the present embodiment having the power supply section 5A is omitted since the observation or the like can be performed by the same procedure as that described in the first embodiment.

As described above, the capsule endoscope 1 of the present embodiment having the power supply section 5A has a configuration in which the capsule endoscope 1 is switched between ON and OFF every time the magnetic field generating section 7 generates the alternating-current magnetic field. Accordingly, the capsule endoscope 1 of the present embodiment having the power supply section 5A can be switched between ON and OFF by applying the alternating-current magnetic field for a very short period of time. As a result, the capsule endoscope 1 of the present embodiment having the power supply section 5A further produces such an effect that burdens on users and test subjects can be reduced in addition to the effect described in the first embodiment.

In the respective embodiments described above, the living body observation system 101 is not limited to the configuration including one magnetic field generating section 7. The living body observation system 101 may also include a plurality of magnetic field generating sections 7.

To be more specific, the living body observation system 101 may include three magnetic field generating sections 7 which are disposed such that directions of generating alternating-current magnetic fields are orthogonal to each other, for example.

When the living body observation system 101 includes the three magnetic field generating sections 7 as described above, it is possible to effectively detect the alternating-current magnetic field in the capsule endoscope 1.

### (Third Embodiment)

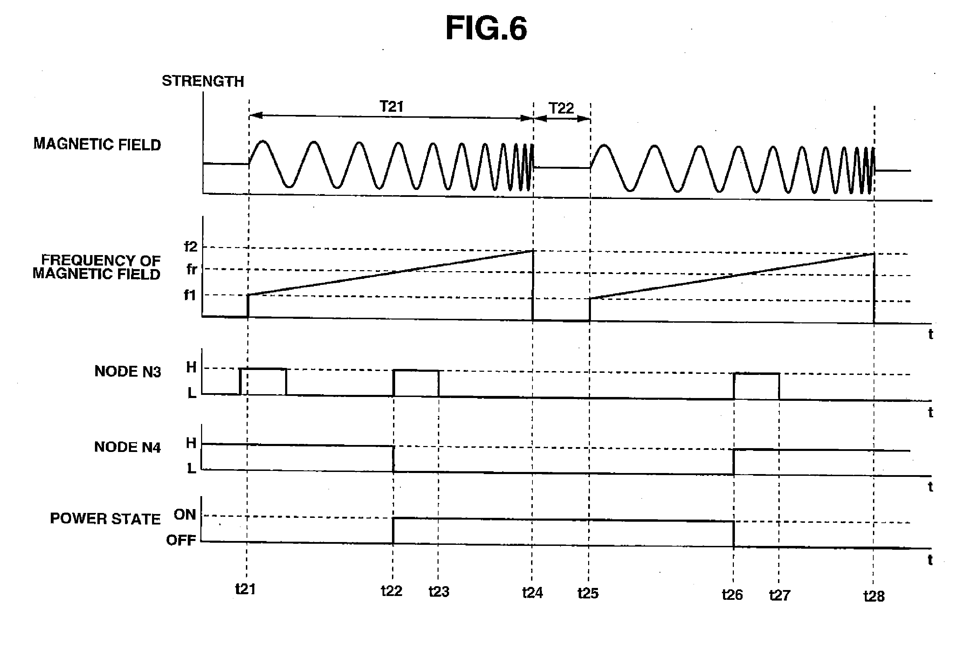

Fig. 6 relates to a third embodiment of the present invention. Fig. 6 illustrates a correlation between operations of a power supply section and a magnetic field detecting section, and a power state of a capsule endoscope according to the third embodiment.

In the following description, the detailed description of portions having the same configurations as those of the first or second embodiment will be omitted. The capsule endoscope of the present embodiment has the same configuration as that of the capsule endoscope of the second embodiment. Therefore, portions different from those of the first and second embodiments will be mainly described in the following.

As shown in Fig. 6, a period from time t21 to time t24 (period T21) is a period when the magnetic field generating section 7 generates an alternating-current magnetic field. Also, as shown in Fig. 6, a period from time t24 to time t25 (period T22) is a period when the magnetic field generating section 7 does not generate an alternating-current magnetic field.

In the present embodiment, the magnetic field generating section 7 generates an alternating-current magnetic field as shown in Fig. 6, that is, a magnetic field whose frequency gradually increases during the period from time t21 to time t24 (period T21). Accordingly, when fr represents a resonant frequency of the resonant circuit constituted by the magnetic field detecting coil 11 and the resonant capacitor 16, f1 represents a sweep lower-limit frequency, and f2 represents a sweep upper-limit frequency, for example, the upper limit and the lower limit of the sweep frequency are determined to satisfy f1 <fr<f2 at all times even when the resonant frequency fr varies.

In the present embodiment, the magnetic field generating section 7 is not limited to generate the magnetic field whose frequency gradually increases during a predetermined period. The magnetic field generating section 7 may also generate a magnetic field whose frequency gradually decreases during the predetermined period.

On the other hand, when the magnetic field whose frequency gradually increases is generated from the magnetic field generating section 7 during the period T21, an alternating-current voltage is generated in the resonant circuit constituted by the magnetic field detecting coil 11 and the resonant capacitor 16 in the vicinity of the resonant frequency fr of the resonant circuit. To be more specific, the alternating-current voltage is generated in the resonant circuit during a period from time t22 to time t23 in Fig. 6.

That is, when the frequency of the alternating-current magnetic field generated from the magnetic field generating section 7 increases with time to approach the resonant frequency fr of the resonant circuit, a potential difference is generated between both ends of the magnetic field detecting coil 11. After that, the frequency of the alternating-current magnetic field generated from the magnetic field generating section 7 exceeds the resonant frequency fr and further increases. When a difference from the resonant frequency fr is larger, the potential difference generated between the both ends of the magnetic field detecting coil 11 disappears.

An electric signal based on the potential difference generated between the both ends of the magnetic field detecting coil 11 passes through the rectifying section 18. Accordingly, the signal level at the node N3 becomes an H level during the period from time t22 to time t23 as shown in Fig. 6. Also, as shown in Fig. 6, when the magnetic field whose frequency gradually increases is generated during a period from time t25 to time t28, the signal level at the node N3 also becomes the H level during a period from time t26 to time t27 in a similar manner.

That is, the magnetic field detecting section 6 of the present embodiment outputs a pulse signal having one pulse every time the alternating-current magnetic field whose frequency gradually increases is generated from the magnetic field generating section 7.

When a pulse signal of the H level having a pulse width of (t23-t22), which is a pulse signal generated during the period from time t21 to time t24, is inputted to the frequency divider circuit 15, the signal level at the node N4 becomes an L level, and also, the P-channel FET 9A becomes an ON state. The power section 8 thus starts to supply driving power to each of the illuminating section 2, the image pickup section 3 and the wireless transmission section 4 to power ON the capsule endoscope 1 during a period from time t22 to time t26.

Also, when a pulse signal of the H level having a pulse width of (t27-t26), which is a pulse signal generated during the period from time t25 to time t28, is inputted to the frequency divider circuit 15, the signal level at the node N4 becomes an H level, and also, the P-channel FET 9A becomes an OFF state. The power section 8 thus stops supplying the driving power to each of the illuminating section 2, the image pickup section 3 and the wireless transmission section 4 to power OFF the capsule endoscope 1 during a period from time t26 to time when the signal level at the node N4 becomes the L level next time.

In the present embodiment, Q value of the resonant circuit constituted by the magnetic field detecting coil 11 and the resonant capacitor 16, and a sweep speed of the frequency of the alternating-current magnetic field generated from the magnetic field generating section 7 are respectively set corresponding to an operation speed of the frequency divider circuit 15.

The description of observation or the like using the capsule endoscope 1 of the present embodiment is omitted since the observation or the like can be performed by the same procedure as that described in the first embodiment.

As described above, the capsule endoscope 1 of the present embodiment can be switched between ON and OFF without adjusting the frequency of the alternating-current magnetic field generated from the magnetic field generating section 7 and the resonant frequency fr of the resonant circuit constituted by the magnetic field detecting coil 11 and the resonant capacitor 16 to each other even when the resonant frequency fr has an individual difference. As a result, the capsule endoscope 1 of the present embodiment further produces such an effect that the capsule endoscope 1 can be easily and stably switched between ON and OFF in addition to the effect described in the first embodiment.

In the case where the living body observation system 101 includes one magnetic field generating section 7, the capsule endoscope 1 is not limited to the configuration including one magnetic field detecting coil 11. The capsule endoscope 1 may also include a plurality of magnetic field detecting coils 11.

To be more specific, the capsule endoscope 1 may include three magnetic field detecting coils 11 which are disposed respectively having effective axes orthogonal to each other such as an x direction corresponding to a front-back direction (long-axis direction) of the capsule endoscope 1, a y direction corresponding to a horizontal direction of the capsule endoscope 1, and a z direction corresponding to a vertical direction of the capsule endoscope 1, for example.

When the capsule endoscope 1 includes the three magnetic field detecting coils 11 as described above, it is possible to improve detection sensitivity to the alternating-current magnetic field generated from the magnetic field generating section 7.

The respective embodiments described above are not only applied to the capsule endoscope, but may be also applied to various living body information acquiring apparatuses having a configuration for acquiring living body information such as a temperature, pH or the like inside a living body.

Also, it goes without saying that the capsule endoscope in each of the embodiments described above can be switched between ON and OFF at a desired timing before and after being disposed in the body of a test subject.

The present invention is not limited to the aforementioned embodiments, and various changes and applications can be made therein without departing from the scope of the invention.

This application claims the benefit of priority from Japanese Patent Application No 2007-263700 filed in Japan on October 9, 2007, disclosed contents of which are cited in this specification, claims, and the drawings.

## Claims

1. A living body information acquiring apparatus comprising:
a living body information acquiring section for acquiring living body information in a living body;
a wireless transmission section for wirelessly transmitting the living body information to outside of the living body;
a power section for supplying driving power of the living body information acquiring section and the wireless transmission section;
a magnetic field detecting section for detecting a magnetic field from outside and outputting a detection result as an electric signal; and
a power supply control section for controlling a supply state of the driving power supplied from the power section to the living body information acquiring section and the wireless transmission section based on the electric signal.

2. The living body information acquiring apparatus according to Claim 1, wherein
the magnetic field detecting section outputs the electric signal when detecting an alternating-current magnetic field from outside.

3. The living body information acquiring apparatus according to Claim 1 or 2, wherein
the power supply control section is a switch section for switching start and stop of the supply of the driving power from the power section to the living body information acquiring section and the wireless transmission section based on the electric signal.

4. The living body information acquiring apparatus according to Claim 3, wherein
the electric signal is a signal for controlling ON and OFF of the switch section.

5. The living body information acquiring apparatus according to Claim 1, wherein
the magnetic field detecting section includes a coil in which a potential difference is generated between both ends in response to an alternating-current magnetic field from outside.

6. The living body information acquiring apparatus according to Claim 1, wherein
the magnetic field detecting section comprises a plurality of magnetic field detecting sections.

7. The living body information acquiring apparatus according to Claim 1, wherein
the living body information acquiring apparatus is a capsule endoscope.

8. A living body observation system comprising:
a living body information acquiring apparatus including a living body information acquiring section for acquiring living body information in a living body, a wireless transmission section for wirelessly transmitting the living body information to outside of the living body, a power section for supplying driving power of the living body information acquiring section and the wireless transmission section, a magnetic field detecting section for detecting a magnetic field from outside and outputting a detection result as an electric signal, and a power supply control section for controlling a supply state of the driving power supplied from the power section to the living body information acquiring section and the wireless transmission section based on the electric signal; and
a magnetic field generating section for generating an alternating-current magnetic field outside the living body information acquiring apparatus.

9. The living body observation system according to Claim 8, wherein
the magnetic field detecting section outputs the electric signal when detecting an alternating-current magnetic field generated from the magnetic field generating section.

10. The living body observation system according to Claim 8 or 9, wherein
the power supply control section is a switch section for switching start and stop of the supply of the driving power from the power section to the living body information acquiring section and the wireless transmission section based on the electric signal.

11. The living body observation system according to Claim 10, wherein
the electric signal is a signal for controlling ON and OFF of the switch section.

12. The living body observation system according to Claim 8, wherein
the magnetic field detecting section includes a coil in which a potential difference is generated between both ends in response to an alternating-current magnetic field generated from the magnetic field generating section.

13. The living body observation system according to Claim 8, wherein
the magnetic field detecting section comprises a plurality of magnetic field detecting sections.

14. The living body observation system according to Claim 8, wherein
the magnetic field generating section comprises a plurality of magnetic field generating sections.

15. The living body observation system according to Claim 8, wherein
the living body information acquiring apparatus is a capsule endoscope.

16. A method of driving the living body observation system recited in Claim 8, wherein
the living body information acquiring apparatus is switched to a power ON state or a power OFF state in response to an alternating-current magnetic field intermittently generated from the magnetic field generating section.

17. A method of driving the living body observation system recited in Claim 8, wherein
the living body information acquiring apparatus is powered ON only during a period when the magnetic field generating section generates an alternating-current magnetic field.

18. A method of driving the living body observation system recited in Claim 8, wherein
the living body information acquiring apparatus is switched to a power ON state or a power OFF state every time the magnetic field generating section generates an alternating-current magnetic field.

19. A method of driving the living body observation system recited in Claim 8, wherein
the living body information acquiring apparatus is switched to a power ON state or a power OFF state in response to an alternating-current magnetic field whose frequency gradually changes, the alternating-current magnetic field being intermittently generated from the magnetic field generating section.

20. The method of driving the living body observation system, according to any one of Claims 16 to 19, wherein
the living body information acquiring apparatus is powered ON outside the living body by applying the alternating-current magnetic field.

21. The method of driving the living body observation system, according to any one of Claims 16 to 19, wherein
the living body information acquiring apparatus is a capsule endoscope.

## Amended claims

### Amended claims under Art. 19.1 PCT

1. (Amended) A living body information acquiring apparatus comprising:
a living body information acquiring section for acquiring living body information in a living body;
a wireless transmission section for wirelessly transmitting the living body information to outside of the living body;
a power section for supplying driving power of the living body information acquiring section and the wireless transmission section;
a magnetic field detecting section for detecting an alternating-current magnetic field from outside and outputting a detection result as an electric signal; and
a power supply control section for controlling a supply state of the driving power supplied from the power section to the living body information acquiring section and the wireless transmission section based on the electric signal.

2. (Canceled)

3. (Amended) The living body information acquiring apparatus according to Claim 1, wherein
the power supply control section is a switch section for switching start and stop of the supply of the driving power from the power section to the living body information acquiring section and the wireless transmission section based on the electric signal.

4. The living body information acquiring apparatus according to Claim 3, wherein
the electric signal is a signal for controlling ON and OFF of the switch section.

5. The living body information acquiring apparatus according to Claim 1, wherein
the magnetic field detecting section includes a coil in which a potential difference is generated between both ends in response to an alternating-current magnetic field from outside.

6. The living body information acquiring apparatus according to Claim 1, wherein
the magnetic field detecting section comprises a plurality of magnetic field detecting sections.

7. The living body information acquiring apparatus according to Claim 1, wherein
the living body information acquiring apparatus is a capsule endoscope.

8. (Amended) A living body observation system comprising:
a living body information acquiring apparatus including a living body information acquiring section for acquiring living body information in a living body, a wireless transmission section for wirelessly transmitting the living body information to outside of the living body, a power section for supplying driving power of the living body information acquiring section and the wireless transmission section, a magnetic field detecting section for detecting an alternating-current magnetic field from outside and outputting a detection result as an electric signal, and a power supply control section for controlling a supply state of the driving power supplied from the power section to the living body information acquiring section and the wireless transmission section based on the electric signal; and
a magnetic field generating section for generating an alternating-current magnetic field outside the living body information acquiring apparatus.

9. (Canceled)

10. (Amended) The living body observation system according to Claim 8, wherein
the power supply control section is a switch section for switching start and stop of the supply of the driving power from the power section to the living body information acquiring section and the wireless transmission section based on the electric signal.

11. The living body observation system according to Claim 10, wherein
the electric signal is a signal for controlling ON and OFF of the switch section.

12. The living body observation system according to Claim 8, wherein
the magnetic field detecting section includes a coil in which a potential difference is generated between both ends in response to an alternating-current magnetic field generated from the magnetic field generating section.

13. The living body observation system according to Claim 8, wherein
the magnetic field detecting section comprises a plurality of magnetic field detecting sections.

14. The living body observation system according to Claim 8, wherein
the magnetic field generating section comprises a plurality of magnetic field generating sections.

15. The living body observation system according to Claim 8, wherein
the living body information acquiring apparatus is a capsule endoscope.

16. A method of driving the living body observation system recited in Claim 8, wherein
the living body information acquiring apparatus is switched to a power ON state or a power OFF state in response to an alternating-current magnetic field intermittently generated from the magnetic field generating section.

17. A method of driving the living body observation system recited in Claim 8, wherein
the living body information acquiring apparatus is powered ON only during a period when the magnetic field generating section generates an alternating-current magnetic field.

18. A method of driving the living body observation system recited in Claim 8, wherein
the living body information acquiring apparatus is switched to a power ON state or a power OFF state every time the magnetic field generating section generates an alternating-current magnetic field.

19. A method of driving the living body observation system recited in Claim 8, wherein
the living body information acquiring apparatus is switched to a power ON state or a power OFF state in response to an alternating-current magnetic field whose frequency gradually changes, the alternating-current magnetic field being intermittently generated from the magnetic field generating section.

20. The method of driving the living body observation system, according to any one of Claims 16 to 19, wherein
the living body information acquiring apparatus is powered ON outside the living body by applying the alternating-current magnetic field.

21. The method of driving the living body observation system, according to any one of Claims 16 to 19, wherein
the living body information acquiring apparatus is a capsule endoscope.
